# EUROPEAN PATENT SPECIFICATION

(11) **EP 1 019 008 B1**
(45) Date of publication and mention of the grant of the patent: **04.05.2005**
(21) Application number: 98944202.5
(22) Date of filing: 20.08.1998
(51) Int. Cl.: A61H 35/00, A61L 2/22, A47K 3/022

(54) **MOBILE AIR-BLAST SPRAYING MACHINE FOR FEET DISINFECTION**
BEWEGLICHE, DRUCKLUFTBETRIEBENE SPRÜHVORRICHTUNG ZUM DESINFIZIEREN DER FÜSSE
MACHINE DE VAPORISATION PAR JETS D'AIR SERVANT A DESINFECTER LES PIEDS

(30) Priority: 29.08.1997 IT MO970148
(43) Date of publication of application: 19.07.2000
(73) Proprietor: Gamar S.R.L., 42015 Correggio (IT)
(72) Inventor: MORA, Giuliano, I-42015 Correggio (IT)
(74) Representative: Guareschi, Antonella
(86) International application number: PCT/IT1998/000235
(87) International publication number: WO 1999/011216

(56) References cited:
- DE-A- 2 745 625
- DE-C- 3 811 517
- US-A- 2 267 169
- US-A- 2 274 739
- US-A- 3 378 009
- US-A- 4 077 546

## Description

### TECHNICAL FIELD

The present invention pertains to the field of machinery and equipment for feet disinfection.

### BACKGROUND ART

As is known, in most swimming pools and bathing establishments fixed basins for feet disinfection are used, for example a brickwork made by digging a ditch or by arranging a raised-step structure, filled with disinfectant liquid, which is often stagnant rather than of a running type. The construction of equipment for feet disinfection is an urging need in many other fields, like for example the medical sector and the food industry, and many other sectors which, however, seldom adopt solutions of the above mentioned kind, since a fixed structure would involve the exclusive and permanent dedication of a predetermined portion of walkable floor, as well as huge maintenance costs, not to mention the appearance, usually not so pleasant, of the known basins.

Are known the priority US-A-2,267,169 and US-A-2,274,739 documents claiming devices destined to the liquid spraying for feet disinfection; both said prior documents are running exclusively mechanical and the cited spraying happens after the press of the user's feet on suitable operating area.

It is also known the US-A-4,077,546 document which claims an appliance suitable to the spraying of sanitized substance for feet where the spraying apparatus is constituted by a common bomb spray; the foot support on an operating area causes the bend or the flexion of itself with consequent crushing of the spray head.

The aim of the present invention is to eliminate the above mentioned drawbacks, while providing a means for feet disinfection, both bare and with footwear, which is easy to install and to maintain, easy to move, safe to use and healthy, as well as aesthetically pleasant and, however, not too expensive.

The stated object is attained with an air-blast spraying machine for feet disinfection, of a mobile or removal type, whose innovative characteristics are illustrated in the claims.

### DISCOLSURE OF THE INVENTION

In a preferred embodiment, the machine according to the present invention can be positioned on the floor in a well-defined passageway, like for example between doorposts or in the middle of a passageway, in order to have people passing by make use of said disinfection machine. In an other embodiment, the machine is particularly suited for wall mounting at a minimum height from the ground, in order to make it easier for the people passing by to use it. Anyway, an advantage of the present invention is that is does not require a permanent connection to a water supply mains or to a drainage system, thus making it possible to use it in isolated or not equipped areas, like for example on the beach.

The air-blast spraying machine can be operated by using actuators of various type, like for example proximity switches, photocells.

Further characteristics and advantages of the present invention will better emerge from the detailed description that follows, made with reference to the accompanying drawings which represent three preferred embodiments in the form of a non limiting example.

The invention will now be described in detail, by way of example, with the aid of the accompanying drawing in which :
- figure 1 shows a perspective view of a first preferred embodiment of the machine according to the present invention;
- figure 2 shows a functional diagram of the component parts of the machine in figure 1;
- figure 3 shows a functional diagram analogous to that of figure 2 of a second embodiment of the machine;

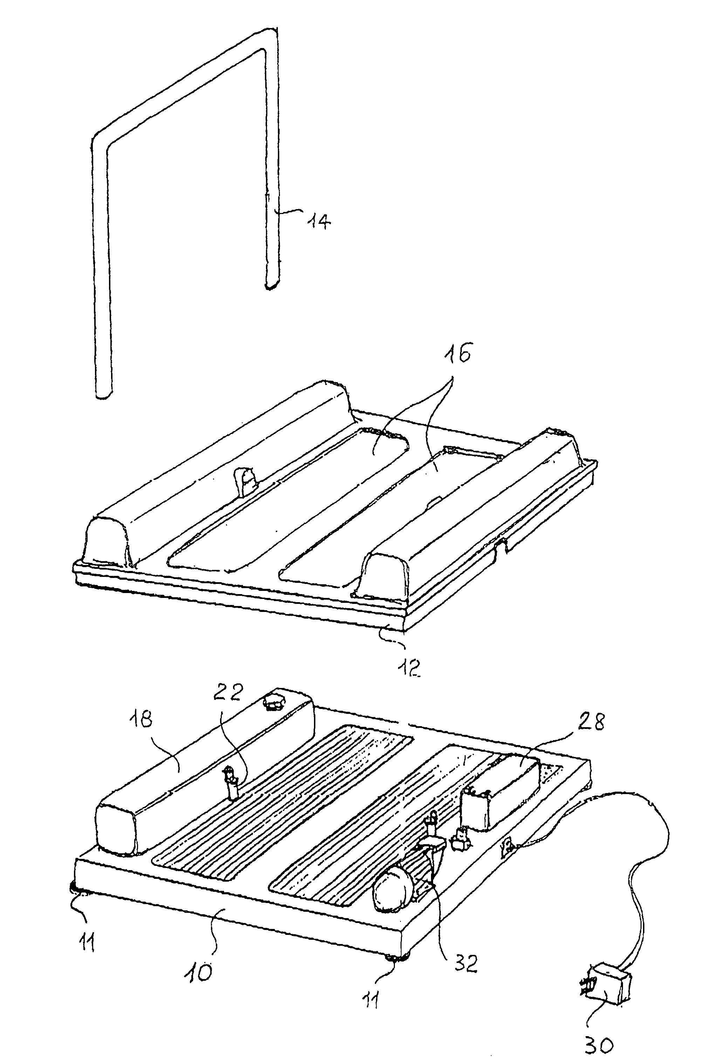

With reference to the figure 1 in the accompanying drawings, an air-blast spraying machine according to the present invention comprises a bearing structure or framework 10, preferably made of, but not limited to, soldered plate, equipped with supporting feet 11, possibly adjustable in height. As an alternative to the feet 11, the bearing structure 10 can be equipped with revolving wheels, at least one of which is preferably fitted with a brake. On the bearing structure 10 a protection cap 12 can be found, functioning as an aesthetically pleasant protective casing, preferably made of, but not limited to, moulded plastic material or fiberglass or the like. To the bearing structure 10 and/or to the cap 12 a bearing handrail 14 can be optionally fixed which can also be used as a handle to move the air-blast spraying machine, especially when this is equipped with wheels.

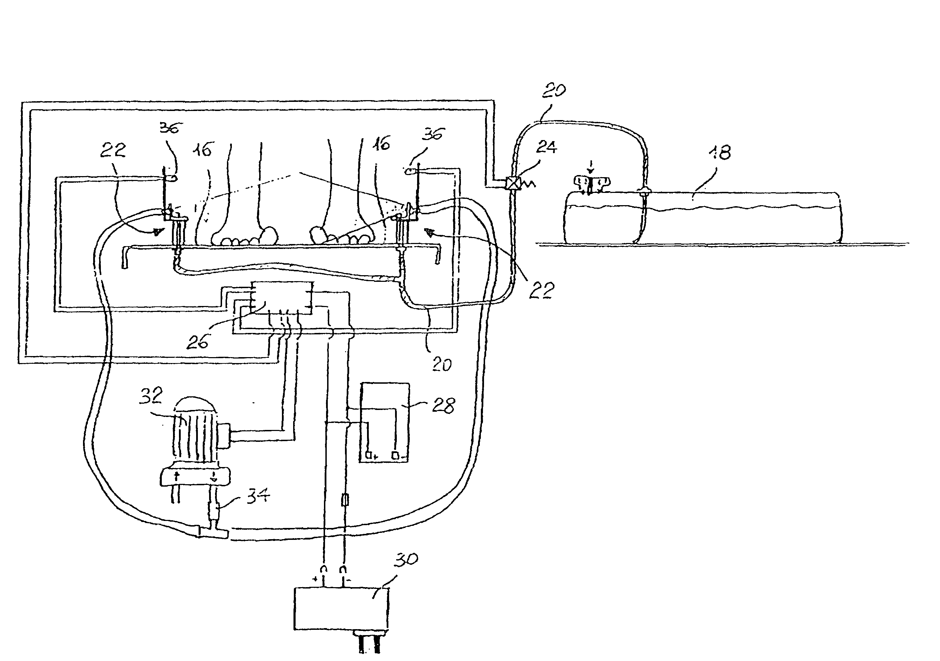

In this central area of the bearing structure 10 or of the cap 12 an operating area 16 is envisaged, said area being constituted by two footboards in the figures 1, 2 and 3, whic is meant to accommodate the feet of the operator of the air-blast spraying machine. On the bearing structure 10, on the sides of the central area occupied by the footboards 16, are placed the elements which are necessary for the operation of the air-blast spraying machine, whose interconnections are better illustrated in figure 2. In particular, from a tank 18 containing disinfectant liquid of a known type protrudes a delivery pipe 20 connected to a couple of air-blast spraying ejectors 22 placed on the sides of the footboards 16. On the pipe 20, a normally closed solenoid valve 24 is normally interposed, whose opening is controlled by a control circuit 26 electrically supplied by a battery 28 fed by a network external feeder 30. The control circuit also selectively controls the operation of the air pump 32 whose delivery pipe 34 is connected to the couple of ejectors 22. A couple of actuators means 36, said means being able to be two proximity switches, also placed on the sides of the footboards 16, is electrically connected to the control circuit 26.

When an operator approaches with his feet one of the two footboards 16, one of the two actuator means 36, said means being constituted by two proximity switches in the figures, sends a presence signal to the control circuit 26 which, in turn, sends an enabling signal meant to open the solenoid valve 24 and simultaneously activates the air pump 32. The liquid contained in the tank 18 is suctioned by the action of the air coming out of the ejectors 22 and there sprayed and directed towards the footboards 16 and, therefore, towards the operator's feet. It has to be noticed that the proximity switches detect the presence of the operator's feet before they come to rest onto the footboards 16, thus providing for the disinfectant to initially reach the foot sole when this is still raised, in order to subsequently disinfect the side and the upper surface of the foot when this eventually comes to rest onto the footboard 16.

As an alternative, the control circuit 26 can also be fitted with a timing device disconnecting the air pump and closing the solenoid valve after a preset time. Although a timing device is not vital to the operation of the object of the present invention, it could however help to avoid the premature emptying of the tank 18 or the useless consumption of electric energy in case of failure of the proximity switches 22.

The presence of the rechargeable battery 28 makes it possible to position the air-blast spraying machine also in a place where a network electric socket is not accessible, which considerably increases the versatility of use of the air-blast spraying machine. Some tests have been made by the Applicant which have shown that a rechargeable battery makes it possible to use the air-blast spraying machine for several days before having to charge the battery again. The use of a rechargeable battery also makes it possible to strategically position the air-blast spraying machine and to install it easily everywhere.

The use of the solenoid valve 24, then, permits to fill the tank 18 above the level of the ejectors 22 in such a way that it is possible to manufacture footboards 16 which are very low with respect to the ground, with the advantages deriving therefrom in term of comfortable use of the air-blast spraying machine.

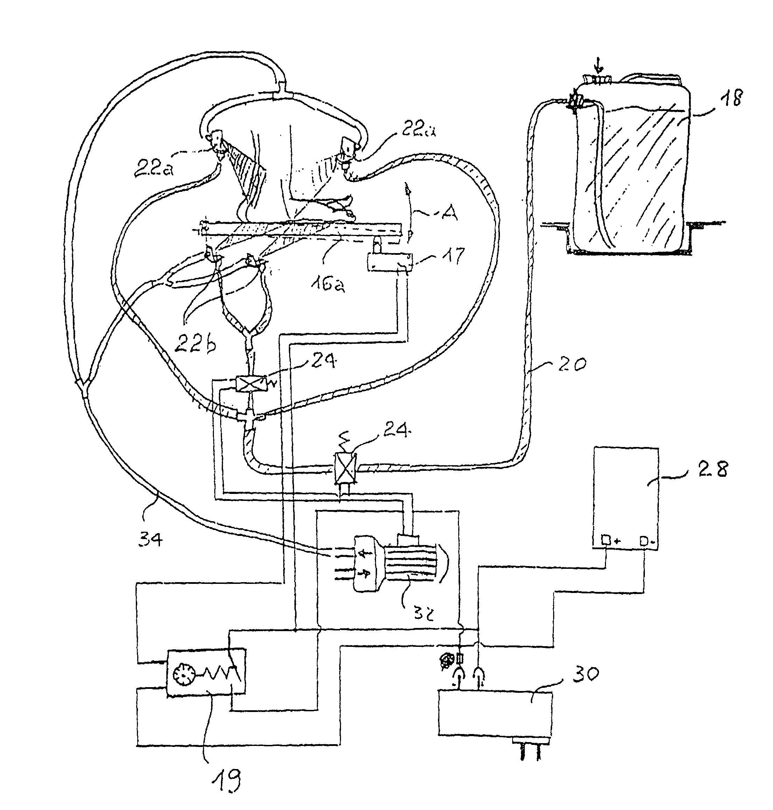

Figure 3 shows a diagram of another embodiment of the air-blast spraying machine object of the present invention, where the elements which are in common with the example described above bear the same reference numbers. The tank 18 containing the disinfectant liquid is connected, by means of the pipe 20, to a first couple of ejectors 22a placed on the sides of a horizontally pivoted footboard 16a, whose oscillating motion according to arrow A, caused by the operator's feet pressure and by the returning action of an elastic element (not illustrated in the figure), activates a switch 17. The articulation of the footboard 16a around the fulcrum 21 can be obtained according to one of the several methods known to experts in the field. A particularly interesting method, thanks to its simple manufacturing and low costs, consists in making an elastic zip directly on the plastic protective casing of the caps of the air-blast spraying machine.

The pipe 20 delivering the disinfectant liquid is also connected to a second couple of ejectors 22b placed under the horizontally pivoted footboard 16a, in such a way that the jet of the sprayed disinfectant liquid is directed onto an operator's feet sole when they are resting on the footboard 16a. In the example of figure 3 a timing circuit 19 is also explicitely illustrated, whose presence is optional.

The machine functioning illustrated in the embodiment of figure 3 is substantially analogous to the above description, with the difference that the presence of an operator's feet is detected by the switch 17, pressed by the horizontally pivoted footboard 16a as a consequence of its rotation downwards, whereas the disconnection of the air pump 32, when it is not determined by the optional timer 19, is caused by the lifting of the horizontally pivoted footboard 16a induced by the elastic return element after the footboard has been abandoned by the operator. Several_embodiments of the above description can be obviously envisaged, all falling within the scope of the present invention. For example, the horizontally pivoted footboard 16a could be replaced by a flexible footboard obtained in the cap 12 made in plastic material thanks to the slightly bombed shape of its central part. Thanks to this solution it is possible to keep the footboard thickness of the air-blast spraying machine at a low level, with the advantages deriving therefrom in terms of comfortable and practical use.

## Claims

1. An air-blast spraying machine for feet disinfection, of a mobile or removable type, comprising a bearing structure (10) containing at least one tank (18) for disinfectant liquid, a covering element comprising at least one operating area (16) for the accommodation of feet to be disinfected, a plurality of air blast ejectors means (22) of the liquid cited connected to the tank and placed in the proximity of the operating area and air pump means (32) electrically charge by a network external feeder (30), **characterised in that** it includes:
- at least one actuator means (36) placed in proximity of the cited operating area, said at least one actuator means being able to reveal the presence of the user's feet nearby of the ejectors means (22) and to selectively control the activation and disactivation of the air pump means (32);
- at least one solenoid valve (24); said solenoid valve being positioned along a delivery pipe (20) of the disinfectant liquid which joins the tank (18) to the ejectors (22);
- a rechargeable battery (28), said battery makes part of a power supply circuit connected to the network external feeding (30).

2. An air-blast spraying machine as claimed in claim 1, **characterised in that** the at least one solenoid valve (24) is of the kind normally closed; said normal configuration of the cited solenoid valve being able to prevent the reflow of the disinfectant liquid when the air-blast spraying machine is not in use.

3. An air-blast spraying machine as claimed in claims 1 and 2, **characterised in that** it comprises a control circuit; the at least one solenoid valve (24) is electrically connected, by the interposition of the control's circuit (26), to the at least one actuator means (36); said connection being able to activate in opening the cited solenoid valve only when the operator's feet presence is intercepted by the cited at least one actuator mean (36).

4. An air-blast spraying machine as claimed in claims 1 and 2, **characterised in that** the disinfectant liquid contained in the tank (18) ist at a higher level with respect to the ejector means (22).

5. An air-blast spraying machine as claimed in claims 1 and 3, **characterised in that** the at least one actuator means (36) is a proximity switch.

6. An air-blast spraying machine as claimed in claim 1, **characterised in that** the rechargeable battery (28) is electrically in parallel to the network external feeder (30).

7. An air-blast spraying machine as claimed in claim 1, **characterised in that** at least one of the ejector means (22) presents an inclination of a spraying nozzle substantially towards the top.

8. An air-blast spraying machine as claimed in claims 1 and 5, **characterised in that** the at least one actuator means is a switch (17) actuable by a pivoted footboard (16a).

## Patentansprüche

1. Fester oder beweglicher Zerstäuber für die Fußdesinfektion, einschließlich einer Halterungsstruktur (10) und mit zumindest einem Tank (18) für die Desinfizierungsflüssigkeit, einem Abdeckelement, ausgestattet mit zumindest einem Betriebsbereich (16) für die Aufnahme der zu desinfizierenden Füße, einer Vielzahl von Ausströmern (22) für die vorgenannte Flüssigkeit, an den Tank angeschlossen und in der Nähe des Betriebsbereichs angebracht, sowie Vorrichtungen zum Pumpen von Luft (32), elektrisch gespeist von einem externen Netzspeiser (30), **dadurch gekennzeichnet, dass** er umfasst:
- zumindest einen Trieb (36) in der Nähe des vorgenannten Betriebsbereiches, wobei dieser Trieb in der Lage ist, das Vorhandensein der Füße des Benutzers in der Nähe der Ausströmer (22) zu erfassen, die Ein- bzw. die Ausschaltung der Vorrichtung (32) für das Pumpen der Luft selektiv zu steuern;
- zumindest ein Magnetventil (24), wobei das vorgenannte Magnetventil am Rohr (20) für den Auslass der Desinfizierungsflüssigkeit angebracht ist, das den Tank (18) mit den Ausströmern (22) verbindet;
- eine nachladbare Batterie (28), wobei die vorgenannte Batterie zur Schaltung der elektrischen Speisung gehört, die an das externe Netzspeiser (30) angeschlossen ist.

2. Zerstäuber gemäß Anspruch 1, **gekennzeichnet durch** die Tatsache, dass zumindest ein Magnetventil (24) ein normalerweise geschlossenes Magnetventil ist; die vorgenannte normale Konfigurierung des vorgenannten Magnetventils ist in der Lage, den Rückfluss der Desinfizierungsflüssigkeit zu unterbrechen, wenn der Zerstäuber nicht in Gebrauch ist.

3. Zerstäuber gemäß den Ansprüchen 1 und 2, **gekennzeichnet durch** die Tatsache, dass er eine Steuerungsschaltung umfasst; zumindest ein Magnetventil (24) ist über die Steuerungsschaltung (26) elektrisch an zumindest einen Trieb (36) angeschlossen; die vorgenannte Steuerungsschaltung ist in der Lage, die Öffnung des vorgenannten Magnetventils nur dann zu öffnen, wenn die Füße des Benutzers von zumindest einem der vorgenannten Triebe (36) erfasst werden.

4. Zerstäuber gemäß den Ansprüchen 1 und 2, **gekennzeichnet durch** die Tatsache, dass die Desinfizierungsflüssigkeit im Tank (18) enthalten ist und sich oberhalb der Ausströmer (22) befindet.

5. Zerstäuber gemäß den Ansprüchen 1 und 3, **gekennzeichnet durch** die Tatsache, dass zumindest ein Trieb (36) ein Näherungsschalter ist.

6. Zerstäuber gemäß Anspruch 1, **gekennzeichnet durch** die Tatsache, dass die nachladbare Batterie (28) elektrisch parallel zum externen Netzspeiser (30) geschaltet ist.

7. Zerstäuber gemäß Anspruch 1, **gekennzeichnet durch** die Tatsache, dass zumindest einer der Ausströmer (22) eine im Wesentlichen nach oben weisende Neigung einer Ausströmerdüse aufweist.

8. Zerstäuber gemäß den Ansprüchen 1 und 5, **gekennzeichnet durch** die Tatsache, dass zumindest ein Trieb ein Schalter (17) ist, der über ein mit einem Scharnier angebrachtes Fußbrett (16a) betätigt wird.

## Revendications

1. Nébuliseur pour la désinfection des pieds, de type fixe ou mobile, comprenant une structure de soutien (10) contenant au moins un réservoir (18) pour le liquide désinfectant, un élément de couverture doté d'au moins une surface opérationnelle (16) destinée à recevoir les pieds à désinfecter, une pluralité d'éjecteurs (22) du dit liquide raccordés au réservoir et disposés à proximité de la surface opérationnelle, ainsi que des dispositifs de pompage de l'air (32) chargés du point de vue électrique par une unité d'alimentation externe de réseau (30), **caractérisé par le fait qu'**il comprend:
- au moins un actionneur (36) situé à proximité de cette surface opérationnelle, cet au moins un actionneur en mesure de détecter la présence des pieds de l'usager à proximité des éjecteurs (22) et de contrôler de façon sélective l'activation et la désactivation des dispositifs de pompage de l'air (32);
- Au moins une électrovanne (24); la dite électrovanne étant située le long d'un tuyau de refoulement (20) du liquide désinfectant qui unit le réservoir (18) aux éjecteurs (22);
- Une batterie rechargeable (28), la dite batterie faisant partie d'un circuit d'alimentation électrique raccordé à l'unité d'alimentation externe de réseau (30).

2. Nébuliseur d'après la revendication 1, **caractérisé par le fait que** l'au moins une électrovanne (24) est de type normalement fermé; la dite configuration normale de la dite électrovanne étant en mesure d'empêcher le reflux du liquide désinfectant lorsque le Nébuliseur n'est pas en fonction.

3. Nébuliseur d'après les revendications 1 et 2, **caractérisé par le fait qu'**il comprend un circuit de contrôle; l'au moins une électrovanne (24) est raccordée du point de vue électrique, par l'intermédiaire du dispositif de contrôle (26), à l'au moins un dispositif actionneur (36); cette connexion étant en mesure d'activer en ouverture la dite électrovanne uniquement si la présence des pieds de l'usager est interceptée par l'au moins un dispositif actionneur (36).

4. Nébuliseur d'après les revendications 1 et 2, **caractérisé par le fait que** le liquide désinfectant contenu dans le réservoir (18) est situé à un niveau supérieur par rapport aux éjecteurs (22).

5. Nébuliseur d'après les revendications 1 et 3, **caractérisé par le fait que** l'au moins un dispositif actionneur (36) est représenté par un interrupteur de proximité.

6. Nébuliseur d'après la revendication 1, **caractérisé par le fait que** la batterie rechargeable (28) est parallèle du point de vue électrique à l'unité d'alimentation externe de réseau (30).

7. Nébuliseur d'après la revendication 1, **caractérisé par le fait qu'**au moins un des éjecteurs (22) présente l'inclinaison d'une buse d'atomisation fondamentalement tournée vers le haut.

8. Nébuliseur d'après les revendications 1 et 5, **caractérisé par le fait que** l'au moins un dispositif actionneur est un interrupteur (17) activable par l'intermédiaire d'une plate-forme montée sur pivot (16a).
